# EUROPEAN PATENT SPECIFICATION

(11) **EP 0 517 820 B1**
(45) Date of publication and mention of the grant of the patent: **21.08.1996**
(21) Application number: 91905921.2
(22) Date of filing: 27.02.1991
(51) Int. Cl.: A61K 31/195

(54) **USE OF 3-GUANIDINOPROPIONIC ACID IN THE MANUFACTURE OF A MEDICAMENT FOR THE TREATMENT AND PREVENTION OF EXCESS ADIPOSITY**
VERWENDUNG VON 3-GUANIDINOPROPIONSÄURE ZUR HERSTELLUNG EINES MEDIKAMENTS ZUR BEHANDLUNG UND PRÄVENTION VON FETTSUCHT
UTILISATION DE L'ACIDE 3-GUANIDINOPROPIONIQUE DANS LA FABRICATION D'UN MEDICAMENT POUR LE TRAITEMENT ET LA PREVENTION DES EXCES D'ADIPOSITE

(30) Priority: 28.02.1990 US 486615; 22.01.1991 WO PCT/US91/00334
(43) Date of publication of application: 16.12.1992
(60) Divisional application: 95117214.7
(73) Proprietor: THE UPJOHN COMPANY, Kalamazoo, Michigan 49001 (US)
(72) Inventor: MEGLASSON, Martin, Durham, Kalamazoo, MI 49002 (US)
(74) Representative: Perry, Robert Edward
(86) International application number: PCT/US91/01109
(87) International publication number: WO 91/12800

(56) References cited:
- GB-A- 1 195 200
- US-A- 3 843 798
- Metabolism, vol. 35, no. 6, june 1986; J.V. Otten et al.: "Thyrotoxics myopathy in mice: accentuation by a creatine transport inhibitor", pages 481-484
- The Biochemical Journal, vol. 232, no. 1, 15 nov. 1985, The Biochemical Society, London, GB; E.A. Shoubridge et al.: "Biochemical adaptation in the skeletal muscle of rats depleted of creatine with the substrate analogue beta-guanidinopropionic acid", pp. 125-131
- The Journal of Clinical Investigation, vol. E61, no. 4, Apr. 1978; D.S. Grosso et al.: "Characterization of a carrier-mediated transport system for taurine in the feta mouse heart in vitro", pp. 944-952

## Description

### Field of the Invention

The present invention relates to a new use of 3-guanidinopropionic acid (3-GPA).

### Background of the Invention

Excess adiposity can be seen in NIDDM (non-insulin-dependent diabetes mellitus) associated with obesity as well as obesity without NIDDM. It is defined as a higher fat body mass-to-lean body mass ratio than that commonly occurring in the general population as measured by whole body specific gravity or other generally accepted means.

3-Guanidinopropionic acid (3-GPA) is an endogenous metabolite found in animals and humans. 3-GPA has been used extensively in the study of creatine metabolism [see, e.g., Walker, Adv. Enzymol. 50:177-242 (1979)] and gamma-aminobutyric acid receptor function [see, e.g., Bowery et al, Br. J. Pharmacol. 50:205-218 (1974)].

Mice fed a diet supplemented with 3-GPA at 20 mg/g and supplied with drinking water containing 5 mg/ml 3-GPA for 7-12 weeks had serum glucose concentrations that did not differ significantly from mice receiving unsupplemented chow and water [see, e.g., Moerland et al, Am. J. Physiol. 257:C810-C816 (1989)].

It is known that in some, but not all cases [see, e.g., Shoubridge et al, Biochem. J. 232:125-131 (1985)], supplementation of the diet with 10-20 mg/g 3-GPA results in decreased body weight [see, e.g., Moerland et al, supra, and Mahanna et al, Exper. Neurol. 68:114-121 (1980)]. This effect has been attributed to decreased skeletal muscle mass and has not been attributed to reduced adiposity or decreased lipid storage [see, e.g., Mahanna et al, supra, and Shields et al, Lab. Invest. 33:151-158 (1975)].

### Summary of the Invention

According to the present invention, it has been found that the use of 3-guanidinopropionic acid or a pharmaceutically-acceptable salt thereof, for the manufacture of a medicament may be effective for use in treating excess adiposity.

Excess adiposity is an etiological factor in NIDDM and when extreme, represents a disease state in itself. 3-GPA decreases adiposity by decreasing the level of lipids stored in fat and liver tissue. The compound is therefore beneficial in the treatment of obesity alone or in concert with NIDDM. The effect of 3-GPA is selective for lipid-rich tissues (e.g. epididymal fat and fatty liver of ob/ob mice) while muscle mass is unaffected or only minimally affected.

Patients having excess adiposity are readily diagnosed by a physician or veterinarian of ordinary skill. By treatment is meant the amelioration or total avoidance of the metabolic disorder as described herein.

For the purposes of the invention, any convenient route of systemic administration is employed, e.g. orally, parenterally, intranasally or intrarectally. In general, the preferred form of administration is orally.

A composition comprising 3-GPA may be administered in a sustained release formulation. By sustained release is meant a formulation in which the drug becomes biologically available to the patient at a measured rate over a prolonged period. Such compositions are well known in the art.

The dosage regimen for 3-GPA in accord with this invention will depend on body weight. 3-GPA, in pharmaceutical dosage form, can range from 1-500 mg/kg/day. The preferred dose is 5-100 mg/kg/day.

The following Examples illustrate the invention.

### Example 1 Improvement in Adiposity

The effect of 3-GPA on body and organ weights is tested in ob/ob mice. The mice receive 3-GPA as a 2 mg/g or 10 mg/g admixture in chow or are fed unsupplemented chow. Data, which is seen in Table 1, is shown as means ±S.E.M.

### Example 2 Reduced Adiposity and Body Weight in Non-diabetic Obese Mice

3-GPA is tested in A^{y} mice obtained from Jackson Laboratories (Bar Harbor, Maine). Mice are determined to be free of glycosuria in the fed state using KetoDiaStix™. 3-GPA is administered for 14 days as an admixture in milled mouse chow at 2 and 5 mg/g chow or unsupplemented chow is provided. Lean and fat body mass is determined using the method of Pace and Rathbun.(See Pace, N. and Rathbun,E.N. Studies on Body Composition. III. The Body Water and Chemically Combined Nitrogen Content Relation to Fat Content. J. Biol. Chem 158:658-691 (1945)). Body weights and the wet weights of excised organs are determined gravimetrically using an analytic laboratory balance.

As shown in Table 2, 3-GPA significantly decreases the body weight and fat mass of mice as percentages of the body weight, diaphragm and calf muscle, which are selected as representative sources of skeletal muscle, are significantly increased by 3-GPA. The total lean body mass (reflecting primarily skeletal muscle mass) is similarly increased by 3-GPA when expressed as a percentage of the body weight.

### Example 3 Effect of 3-GPA on Insulin Sensitivity and Body Weight in Adult Rhesus Monkeys

3-GPA is administered orally in gelatin capsule three times daily at a dose of 16 mg/kg for 11 days. Insulin sensitivity is determined using the Bergman Minimal Model technique (Pacini, G. and Bergman, R.N:MINMOD:a computer program to calculate insulin sensitivity and pancreatic responsivity from the frequently sampled intravenous glucose tolerance test, Computer Meth. Progr. Biomed. 23:113-122, 1986). Insulin sensitivity was improved in all subjects. Body weight decreased in the two heaviest monkeys, but was unaffected in a low body weight monkey. These findings are consistent with previous data from obese rodents where 3-GPA decreased body weight by preferentially lowering the body fat mass without affecting the lean tissue mass and increased insulin sensitivity.

**Table 1**

| Experiment 1. 31 days treatment. 4-6 mice/group | | | |
|---|---|---|---|
| Concentration in Chow: | 0 | 2 mg/g | 10 mg/g |
| Body Weight (g) | 52.3+0.7 | 49.4+1.1 | 36.1+1.5 |
| Liver (g) | 4.3+0.2 | 3.6+0.1 | 1.6+0.2 |
| Epididymal Fat (g) | 3.7+0.1 | 3.8+0.3 | 2.5+0.2 |

| Experiment 2. 13 days treatment. 5 mice/group | | | |
|---|---|---|---|
| Concentration in Chow: | 0 | 10 mg/g | |
| Body Weight (g) | 43.4+1.3 | 36.8+1.3 | |
| Liver (g) | 2.4+0.2 | 1.3+0.1 | |
| Heart (g) | 0.11+0.01 | 0.10+0.004 | |
| Diaphragm (g) | 0.065+0.004 | 0.061+0.004 | |
| Calf muscle (g) | 0.050+0.001 | 0.047+0.003 | |

**Table 2**

| Addition of 3-GPA to Chow (mg/g): | | | |
|---|---|---|---|
| | 0 (n=4) | 2 (n=5) | 5 (n=5) |
| Body Weight (g) | | | |
| Initial | 42.72±1.19 | 43.55±1.09 | 43.02±0.82 |
| Final | 45.70±1.64 | 45.53±0.97 | 34.97±1.16*** |
| Fat Mass (g) | 13.45±0.69 | 13.87±0.38 | 8.65±0.81** |
| Lean Body Mass (% BW) | 70.60±0.78 | 69.54±0.57 | 75.47±1.57* |
| Diaphragm | | | |
| (mg) | 97.5±2.7 | 103.9±1.2 | 96.4±4.2 |
| (% BW) | 0.21±0.01 | 0.23±0.004 | 0.28±0.018** |

| Calf Muscle | | | |
|---|---|---|---|
| (mg) | 133.5±23.0 | 143.2±5.6 | 142.5±4.2 |
| (%BW) | 0.29±0.05 | 0.32±0.018 | 0.41±0.018* |
| Data are shown as means ± S.E.M. Statistical analysis was performed by analysis of variance. P-values for comparison to control mice: *, P<0.05. **, P<0.01. ***, P<0.001. | | | |

## Claims

1. Use of 3-guanidinopropionic acid or a pharmaceutically-acceptable salt thereof, for the manufacture of a medicament for use in treating excess adiposity.

2. Use according to claim 1, wherein the medicament is adapted for oral administration.

3. Use according to claim 1 or claim 2, wherein the acid or salt is administered in admixture with the diet.

4. Use according to any of claims 1 to 3, wherein the excess adiposity is associated with NIDDM.

5. Use according to any of claims 1 to 3, wherein the excess adiposity is not associated with NIDDM.

## Patentansprüche

1. Verwendung von 3-Guanidinopropionsäure oder eines pharmazeutisch akzeptablen Salzes derselben zur Herstellung eines Medikaments zur Verwendung bei der Behandlung von übermäßiger Fettsucht.

2. Verwendung nach Anspruch 1, wobei das Medikament zur oralen Verabreichung ausgelegt ist.

3. Verwendung nach Anspruch 1 oder 2, wobei die Säure oder das Salz in Mischung mit der Nahrung verabreicht wird.

4. Verwendung nach einem der Ansprüche 1 bis 3, wobei die übermäßige Fettsucht mit NIDDM vergesellschaftet ist.

5. Verwendung nach einem der Ansprüche 1 bis 3, wobei die übermäßige Fettsucht nicht mit NIDDM vergesellschaftet ist.

## Revendications

1. Utilisation d'acide 3-guanidinopropionique ou d'un de ses sels pharmaceutiquement acceptables pour la production d'un médicament destiné à être utilisé dans le traitement d'une adiposité excessive.

2. Utilisation suivant la revendication 1, dans laquelle le médicament est apte à l'administration par voie orale.

3. Utilisation suivant la revendication 1 ou la revendication 2, dans laquelle l'acide ou le sel est administré en mélange avec le régime alimentaire.

4. Utilisation suivant l'une quelconque des revendications 1 à 3, dans laquelle l'adiposité excessive est associée à un diabète non insulino-dépendant (NIDDM).

5. Utilisation suivant l'une quelconque des revendications 1 à 3, dans laquelle l'adiposité excessive n'est pas associée à un NIDDM.
